# EUROPEAN PATENT SPECIFICATION

(11) **EP 2 672 859 B1**
(45) Date of publication and mention of the grant of the patent: **16.05.2018**
(21) Application number: 12705443.5
(22) Date of filing: 08.02.2012
(51) Int. Cl.: A45D 19/02, A45D 34/04

(54) **PROCESS FOR APPLYING A HAIR CARE COMPOSITION TO HAIR**
VERFAHREN ZUM AUFBRINGEN EINES HAARBEHANDLUNGSMITTELS AUF DIE HAARE
PROCÉDÉ D'APPLICATION D'UNE COMPOSITION DE SOINS CAPILLAIRES SUR LES CHEVEUX

(30) Priority: 08.02.2011 FR 1151005; 09.03.2011 US 201161450826 P
(43) Date of publication of application: 18.12.2013
(73) Proprietor: L'Oréal, 75008 Paris (FR)
(72) Inventor: SAMAIN, Henri, F-91570 Bievres (FR); GAWTREY, Jonathan, F-92100 Boulogne (FR)
(74) Representative: Nony
(86) International application number: PCT/IB2012/050566
(87) International publication number: WO 2012/107887

(56) References cited:
- GB-A- 2 419 089
- US-A- 5 761 824
- US-A1- 2006 247 585

## Description

The present invention relates to treatments for keratinous substances or mucous membranes. The present invention relates in particular, but not exclusively, to styling and shaping hair.

Application US 2006/247585 discloses disposal applicators in the shape of hand or finger mitts, gloves, flat or tubular pads, wraps, facemasks, booties, to manually apply controllable amounts of lotions, creams, gels or dry powders onto target skin and hard surfaces.

The most widespread devices for shaping and/or setting hair are aerosol systems that deliver droplets of a hair care product propelled by a gas, generally liquefied, based upon an alcoholic or aqueous solution and polymer resins, forming fine joins between hairs when dry.

The use of a liquefied gas leads to production, use and recycling constraints.

What is more, the jet of an aerosol is not always easy to direct on the hair and spraying generally causes loss of product, even stains, in particular to clothing, and risks of projection onto the face, for example in the eyes, which can be a substantial problem.

It is further difficult to obtain both good styling properties and satisfactory lacquering power, since some lacquers give hair for example, in particular dyed hair, a dry feel.

What is more, some setting compounds are in a usual semi-solid, viscous or powdery form, and it is difficult to implement conventional processes with them, in particular to ensure an even distribution on all the hair to be treated.

Accordingly, some products with potentially promising setting power from a hair care point of view are not used, or hardly used, in the form of spray for reasons of incompatibility with such a distribution form.

Patent US 5 761 824 discloses a moisturizing attachment for use with a conventional hand held hair dryer. The attachment includes a hollowed interior holding compartment for storing a quantity of a moisturizing means, as pebbles, able to trap volume of the fluid in the spaces between them.

Application GB 2 419 089, describes a method of hair treatment using a foraminous bag containing a fibrous or leaf like material suitable for treatment of hair; and a second hair treating material deposited on the outside surface of the bag.

There is a need to remedy the drawbacks of existing devices.

There is also a need for a device compatible with a large range of hair care compositions.

The invention aims to meet at least one of these needs.

The invention relates to a process of holding the hair style by depositing a composition comprising a setting agent on the surface of hair.

According to a first feature, the subject of the present invention is a process for styling hair as defined in claim 1 of the present application. The process is for example a process for styling and treating hair, the composition being a hair care composition. This type of application does not destroy the hair style and, without pressing or rubbing the support on the hair, delivers a setting effect of very high quality, equivalent for example to the result obtained with a conventional aerosol lacquer, without its drawbacks. In particular, it distributes the product for hair care treatment well.

In what follows, it is understood that the support constitutes an application device or belongs to an application device.

By virtue of the invention, the composition can be applied precisely, without dripping. The invention gives hair natural and long-lasting hold. The characteristics of the droplets that are deposited onto the hair can easily be controlled, in particular their size, by selecting the sizes of the cavities: finer droplets give the hair a lighter, more natural feel, whereas droplets with higher diameter give a stronger hold. The characteristics of the support may be adapted depending on the type of application targeted, in particular weaker or stronger styling, hair design or setting, for example by selecting higher or lower cavity density and/or selecting the material of which the support is made. The surface tensions observed in the cavities depend on the material selected. If the surface is perfectly hydrophobic, it is difficult or even impossible to create droplets of water or aqueous composition that have satisfactory size. If the surface is very hydrophilic, the droplets cannot be transferred easily. The material of the support may create different surface tensions depending on the cavities; these mean that some cavities only trap some elements of the composition and that others trap different elements.

Each cavity in the support may contain one droplet. The largest opening size, in particular the diameter of each cavity, varies preferably from 30 µm to 3 mm and, so that the droplet released by the cavity has a volume varying from 0.0001 µl to 10 µl and more particularly from 0.001 µl to 3 µl.

The cavities may not communicate between themselves, which is in particular the case when the cavities are formed by the mesh of a grid or when they are formed at the tip of studs or pins.

The support may present portions in relief comprising the cavities. For example the cavities form hollow tips of portions in relief. These hollow tips may have a semispherical shape.

The number of cavities may vary from 1 to 100,000, preferably from 50 to 50,000, more preferably from 100 to 5,000, the cavities extending for example on a surface area of the support varying from 1 to 1,000 cm², preferably from 5 to 500 cm².

The process according to the invention may be implemented on dry hair or wet hair. The hair is preferably dry.

There are several ways to transfer the droplets of composition from the support to the hair.

The support may be brought into contact with the hair. The droplets would then be transferred to the hair by capillary action.

In another variant, in addition to putting the support in contact with the hair, a mechanical action, exerted for example by the user, contributes to the ejection of droplets.

In a variant, the support is not put in contact with the hair but is placed at a distance close enough, for example less than 20 cm, preferably less than 5 cm, and the droplets are ejected by a mechanical action.

"Mechanical action" is understood to denote any action by which the droplets are subjected to a physical phenomenon causing them to move from the cavities towards the hair. The mechanical action may be an acceleration of the support and/or a variation of pressure in the area around the droplet or a perturbation such as a vibration. This may be a modification to the shape of the cavity.

When the cavities allow droplets to be ejected on demand, the cavities are qualified as active cavities.

The mechanical action may be caused by a source of vibrations, for example a piezoelectric element or a centrifugal weight, by a pressure source, or a thermal or electric phenomenon. The mechanical action may be generated by an ejection system belonging to the device, for example integrated into the support or coupled to it.

The support is for example subjected to an acceleration due to the user tapping the support, for example when it is located near or in contact with the hair, which has the effect of depositing the drops of product on the hair, or to vibrations generated by a vibrating source.

The support may be subjected, for example on an opposite face compared to the application face facing the hair, to a pressure increase due to a gas, for example air blown through the support.

The droplets may be ejected using a gas jet, preferably coming from a container of compressed air.

The droplet of composition contained in a cavity may also be subjected to a bubble of vapor obtained by heating the composition, this bubble of vapor causing, by slackening, the ejection of the droplet.

According to the embodiments, the cavities of the support may cross through it or not. Cavities that cross through are preferred when the ejection of droplets and/or their transfer to hair is caused or assisted by a gas, preferably air, blown behind the support in the direction of the hair.

After transferring the composition to the hair, the support may be withdrawn and the droplets of composition may be dried on the hair, for example using a hair dryer or helmet dryer, or they may be left to dry naturally.

In an example of implementation of the invention, the support, placed in contact with the hair or held at a distance from the hair, is subjected to an air jet.

By using an air jet, the droplets may be transferred and dried on the hair almost simultaneously. The air jet, allowing or facilitating the droplets of composition to be transferred onto the hair, may be a jet of hot, cold or room-temperature air. The air jet may be produced by a source of compressed air or the blowing from a hair dryer or helmet dryer.

The composition may be transferred by exerting several combined actions, for example by tapping the support while subjecting it to a puff of gas.

The support may be configured to store the droplets of composition and eject them upon demand. In other words, the device may be controlled to eject the droplets automatically, the ejection taking place for example from all the cavities or only some cavities, in the case where the device allows the ejection by one cavity to be controlled individually.

Ejection upon demand of droplets may result from a thermal effect or use of a piezoelectric element.

In an example of implementation, the device makes it possible to heat the composition, in particular within a cavity to cause it to vaporize and generate an excess pressure that causes a droplet to be ejected and transferred to the hair. This technique is related to the thermal process for the ink-jet printing technique, and may be used to cause the composition to be transferred on demand selectively.

The support may integrate one or more electric resistances that vaporize the composition.

The droplets may also be ejected from the support by a piezoelectric element.

The use of a piezoelectric element may allow ejection on demand, this piezoelectric element optionally being combined with one or more specific cavities.

In one example, the interior volume of the cavities varies under the effect of one or more piezoelectric elements, and droplets are ejected and transferred to the hair. This technique is related to the piezoelectric process for the ink-jet printing technique.

The device according to the invention, whether or not it is made for single use, may be devoid of any electric component, so as to be made at low cost. Accordingly, in examples of implementation, the support may comprise a simple mesh made of a non-absorbent material.

### Charging the support with the composition

The process according to the invention may comprise a prior step consisting in loading the support with the hair care composition to be applied.

Several methods of loading the support with the composition are possible, depending on the goal targeted, in particular styling, hair design or setting.

The support may be supplied with composition, or loaded with composition before each use.

The support may be supplied with the composition automatically or not. The support may be part of a device that comprises a reservoir containing the composition to be applied. The cavities may each communicate, if need be, with the reservoir.

The composition may be sampled directly by the support from a container containing the composition. The support may for example be pressed onto a sponge soaked with composition, so as to be loaded with composition. As a variant, the support is submerged in the composition. The support may also be brought into contact with a roll loaded with composition.

Preferably, the support may be cleaned after use and the composition remaining on the support after use may be removed easily, for example being cleaned with water or using any suitable solvent, by using an absorbent cloth, by aspiration or by gas jet, this list not being limiting.

The fact that the support may be reloaded with composition means it may be reused. The support is for example reloaded with a composition identical to the composition used previously, or the support may be loaded with a different composition.

The composition being loaded on the support may involve the excess composition on the support being removed, so that the composition is not present anywhere other than in the cavities. For example, the support may be brought into contact with a scraper after immersion in a reservoir containing the composition, this scraper being for example formed by the free edge of one wall of the container containing the composition.

When the support comprises tips or studs carrying at their ends the cavities, the support is loaded by bringing the tips in contact with the composition.

### Styling and/or setting cosmetic compositions

The compositions that may be used in the present invention are in the form of liquids or gel creams, pastes, comprising or not comprising a granular or powder phase. When the composition is liquid, it may be in the form of lotion or emulsion

Preferably, the composition is in the form of a liquid, a lotion or a fluid emulsion or a gel that is not very thick.

The viscosity of the composition varies preferably from 1 and 200 cps at 25°C and at a shear rate of 1s⁻¹.

The viscosity measurements to which reference is made in the present application are measured using a rheometer with cone-plate geometry.

In particular, within the scope of the present invention, a composition known to the person skilled in the art for styling and setting hair may be selected and in particular, those added to aerosol devices in the presence of a propellant gas to be used in lacquer form. In accordance with the invention, the composition comprises at least one setting agent, as needed in a cosmetically acceptable medium, this medium preferably being water-, alcohol- or aqueous alcohol-based.

The cosmetic composition according to the invention may also comprise one or more organic solvents, preferably in an amount between 0.05 and 95% weight, very preferably between 1 and 70% by weight, relative to the total weight of the composition.

This organic solvent may be a C₂ to C₄ lower alcohol, in particular ethanol and isopropanol, polyols and polyol ethers such as propylene glycol, polyethylene glycol or glycerol. The organic solvent is preferably ethanol or isopropanol, and even more preferably is ethanol.

### Fixing polymer

The composition comprises one or more fixing polymers as setting agents.

The expression "fixing polymer" is understood within the meaning of the present invention to be any polymer that makes it possible to give a shape to the hair or to hold the hair in a given shape.

All the anionic, cationic, amphoteric and nonionic fixing polymers and mixtures thereof used may be used as fixing polymers in the compositions according to the present application.

The fixing polymers may be soluble in the cosmetically acceptable medium or insoluble in this same medium and used in this case in the form of dispersions of solid or liquid particles of polymer (latex or pseudolatex). The anionic fixing polymers generally used are polymers comprising groups derived from carboxylic, sulfonic or phosphoric acids and have an average molecular weight by number of between about 500 and 5,000,000. The carboxylic groups are provided by unsaturated mono- or diacid carboxylic monomers such as those that have the formula: 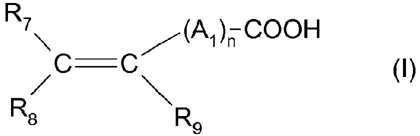 in which n is an integer from 0 to 10, A₁ denotes a methylene group optionally joined to the carbon atom of the unsaturated group or to the adjacent methylene group when n is greater than 1, via a heteroatom such as oxygen or sulfur, R₇ denotes a hydrogen atom or a phenyl or benzyl group, R₈ denotes a hydrogen atom or a lower alkyl or carboxyl group, and R₉ denotes a hydrogen atom, a lower alkyl group, a CH₂-COOH, phenyl or benzyl group.

In the abovementioned formula, a lower alkyl group preferably denotes a group containing 1 to 4 carbon atoms and in particular methyl and ethyl groups. The anionic fixing polymers containing carboxylic groups that are preferred according to the invention are:
A) Copolymers of acrylic or methacrylic acid or salts thereof, and in particular copolymers of acrylic acid and acrylamide sold in the form of their sodium salts as Reten 421, 423 or 425 by Hercules;
B) Copolymers of acrylic or methacrylic acid with a monoethylenic monomer such as ethylene, styrene, vinyl esters and acrylic or methacrylic acid esters, optionally grafted onto a polyalkylene glycol such as polyethylene glycol and optionally crosslinked. Such polymers are described in particular in French patent 1 222 944 and German patent application 2 330 956, the copolymers of this type comprising an optionally N-alkylated and/or hydroxyalkylated acrylamide unit in their chain as described in particular in Luxembourg patent applications 75370 and 75371 or sold as Quadramer by American Cyanamid. Mention may also be made of the acrylic acid/ethyl acrylate/N-*tert-*butylacrylamide terpolymers such as Ultrahold Strong sold by BASF. Mention may also be made of copolymers of acrylic acid and C₁-C₄ alkyl methacrylate and terpolymers of vinylpyrrolidone, acrylic acid and C₁-C₂₀ alkylmethacrylate, for example lauryl methacrylate, such as the product sold by ISP as Acrylidone® LM and methacrylic acid/ethyl acrylate/*tert*-butyl acrylate terpolymers such as the product sold as Luvimer® 100 P by BASF.
   Mention may also be made of methacrylic acid/acrylic acid/ethyl acrylate/methyl methacrylate copolymers in an aqueous dispersion, sold as Amerhold® DR 25 by Amerchol;
C) Crotonic acid copolymers, such as those comprising vinyl acetate or propionate units in their chain and optionally other monomers such as allyl esters or methallyl esters, vinyl ether or vinyl ester of a linear or branched saturated carboxylic acid with a long hydrocarbon-based chain, such as those containing at least 5 carbon atoms, it being possible for these polymers optionally to be grafted or crosslinked, or alternatively another vinyl, allyl or methallyl ester monomer of an [alpha]- or [beta]-cyclic carboxylic acid. Such polymers are described, *inter alia*, in French patents 1 222 944, 1 580 545, 2 265 782, 2 265 781, 1 564 110 and 2 439 798. Commercial products that come under this category are the resins 28-29-30, 26-13-14 and 28-13-10 sold by National Starch;
D) Copolymers of C₄-C₈ monounsaturated carboxylic acids or anhydrides selected from: copolymers comprising (i) one or more maleic, fumaric or itaconic acids or anhydrides and (ii) at least one monomer selected from vinyl esters, vinyl ethers, vinyl halides, phenylvinyl derivatives, acrylic acid and its esters, the anhydride functions of these copolymers optionally being monoesterified or monoamidated. Such polymers are described, in particular, in US patents 2 047 398, 2 723 248 and 2 102 113, and GB patent 839 805. Commercial products are in particular those sold as Gantrez® AN or ES by ISP; copolymers comprising (i) one or more maleic, citraconic or itaconic anhydride units and (ii) one or more monomers selected from allyl or methallyl esters optionally comprising one or more acrylamide, methacrylamide, [alpha]-olefin, acrylic or methacrylic ester, acrylic or methacrylic acid or vinylpyrrolidone groups in their chain, the anhydride functions of these copolymers optionally being monoesterified or monoamidated. These polymers are described, for example, in French patents 2 350 384 and 2 357 241 of the applicant.
E) Polyacrylamides comprising carboxylate groups.
F) Homopolymers and copolymers comprising sulfonic groups such as polymers comprising vinylsulfonic, styrenesulfonic, naphthalenesulfonic or acrylamidoalkylsulfonic units, different from the branched sulfonic polyesters of the invention. These polymers may in particular be selected from: salts of polyvinylsulfonic acid having a molecular weight between about 1,000 and 100,000, and copolymers with an unsaturated comonomer such as acrylic or methacrylic acids and esters thereof, and acrylamide or derivatives thereof, vinyl ethers and vinylpyrrolidone; salts of polystyrenesulfonic acid such as sodium salts sold for example as Flexan® 500 and Flexan® 130 by National Starch. These compounds are described in French patent 2,198,719; polyacrylamidesulfonic acid salts, such as those mentioned in US patent 4 128 631 and more particularly polyacrylamidoethylpropanesulfonic acid sold as Cosmedia Polymer HSP 1180 by Henkel.

As another anionic setting polymer that may be used according to the invention, mention may be made of the branched block anionic polymer sold as Fixate G-100 by Noveon. According to the invention, the anionic fixing polymers are preferably selected from copolymers of acrylic acid or of acrylic esters, such as the acrylic acid/ethyl acrylate/N-*tert*-butylacrylamide terpolymers sold especially as Ultrahold® Strong by BASF, copolymers derived from crotonic acid, such as vinyl acetate/vinyl *tert-*butylbenzoate/crotonic acid terpolymers and the crotonic acid/vinyl acetate/vinyl neododecanoate terpolymers sold especially as Resin 28-29-30 by National Starch, polymers derived from maleic, fumaric or itaconic acids or anhydrides with vinyl esters, vinyl ethers, vinyl halides, phenylvinyl derivatives and acrylic acid and esters thereof, such as the methyl vinyl ether/monoesterified maleic anhydride copolymers sold, for example, as Gantrez® by ISP, the copolymers of methacrylic acid and methyl methacrylate sold as Eudragit® L by Rohm Pharma, the copolymers of methacrylic acid and ethyl acrylate sold as Luvimer® MAEX or MAE by BASF, the vinyl acetate/crotonic acid copolymers sold as Luviset CA 66 by BASF, the vinyl acetate/crotonic acid copolymers grafted with polyethylene glycol sold as Aristoflex® A by BASF, and the polymer sold as Fixate G-100 by Noveon.

Among the anionic fixing polymers mentioned above, it is more particularly preferred in the context of the present invention to use the methyl vinyl ether/monoesterified maleic anhydride copolymers sold as Gantrez® ES 425 by ISP, the acrylic acid/ethyl acrylate/N-*tert*-butylacrylamide terpolymers sold as Ultrahold® Strong by BASF, the copolymers of methacrylic acid and methyl methacrylate sold as Eudragit® L by Rohm Pharma, the vinyl acetate/vinyl *tert*-butylbenzoate/crotonic acid terpolymers and the crotonic acid/vinyl acetate/vinyl neododecanoate terpolymers sold as Resin 28-29-30 by National Starch, the copolymers of methacrylic acid and ethyl acrylate sold as Luvimer® MAEX or MAE by BASF, the vinylpyrrolidone/acrylic acid/lauryl methacrylate terpolymers sold as Acrylidone® LM by ISP and the polymer sold as Fixate G-100 by Noveon.

The cationic setting film-forming polymers that may be used according to the present invention are preferably selected from polymers comprising primary, secondary, tertiary and/or quaternary amine groups forming part of the polymer chain or directly attached thereto, and having preferably a molecular weight of between 500 and about 5,000,000 and preferably between 1,000 and 3,000,000. Among these polymers, mention may be made more particularly of the following cationic polymers:
(1) homopolymers or copolymers derived from acrylic or methacrylic esters or amides and comprising at least one of the units of the following formulae: 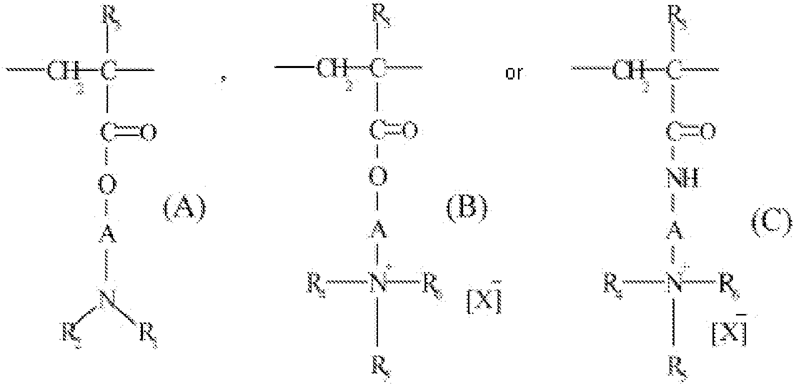 in which: R3 designates a hydrogen atom or a CH3 radical; A is a linear or branched alkyl group comprising from 1 to 6 carbon atoms or a hydroxyalkyl group comprising from 1 to 4 carbon atoms; R4, R5, R6, which may be identical or different, represent an alkyl group having from 1 to 18 carbon atoms or a benzyl radical; R1 and R2, which may be identical or different, each represent a hydrogen atom or an alkyl group having from 1 to 6 carbon atoms; X designates a methosulfate anion or a halide such as chloride or bromide. Copolymers from the family (1) further contain one or more units derived from comonomers that may be selected from the family of acrylamides, methacrylamides, diacetones-acrylamides, acrylamides and methacrylamides substituted on the nitrogen by lower alkyl groups (C₁-C₄), groups derived from acrylic or methacrylic acids or esters thereof, vinyllactams such as vinylpyrrolidone or vinylcaprolactam, and vinyl esters. Accordingly, among these copolymers from family (1), mention may be made of: copolymers of dimethylaminoethyl acrylamide and methacrylate quaternized with dimethyl sulfate or with a dimethyl halide, such as that sold as Hercofloc® by Hercules, copolymers of acrylamide and methacryloyloxyethyltrimethylammonium chloride described for example in patent application EP-A-080976 and sold as Bina Quat P 100 by Ciba Geigy, the copolymer of acrylamide and methacryloyloxyethyltrimethylammonium methosulfate such as that sold as Reten by Hercules, vinylpyrrolidone/dialkylaminoalkyl acrylate or methacrylate copolymers, quaternized or not, such as the products sold as Gafquat® by ISP such as for example Gafquat® 734 or Gafquat® 755 or the products called Copolymer® 845, 958 and 937. These polymers are described in detail in French patents 2 077 143 and 2 393 573, fatty-chain polymers and with vinylpyrrolidone units, such as the products sold as Stylèze W20 and Stylèze W10 by ISP, dimethylaminoethyl methacrylate/vinylcaprolactam/vinylpyrrolidone terpolymers such as the product sold as Gaffix VC 713 by ISP, and quaternized vinylpyrrolidone/dimethylaminopropyl methacrylamide copolymers such as the products sold as Gafquat® HS 100 by ISP.
(2) non-cellulosic cationic polysaccharides, preferably containing quaternary ammonium, such as those described in US patents 3 589 578 and 4 031 307, such as guar gums containing trialkylammonium cationic groups. Such products are sold in particular under the trade names Jaguar C13 S, Jaguar C 15 and Jaguar C 17 by Meyhall;
(3) quaternary copolymers of vinylpyrrolidone and of vinylimidazole;
(4) chitosans or salts thereof; the salts that may be used are, in particular, chitosan acetate, lactate, glutamate, gluconate or pyrrolidonecarboxylate. Among these compounds, mention may be made of chitosan having a degree of deacetylation of 90.5% by weight, sold as Kytan Brut Standard by Aber Technologies, and chitosan pyrrolidonecarboxylate sold as Kytamer® PC by Amerchol.
(5) cationic cellulose derivatives such as copolymers of cellulose or of cellulose derivatives grafted with a water-soluble monomer comprising a quaternary ammonium, and disclosed in particular in US patent 4 131 576, such as hydroxyalkylcelluloses, for example hydroxymethyl-, hydroxyethyl- or hydroxypropylcelluloses grafted in particular with a methacryloyloxyethyltrimethylammonium, methacrylamidopropyltrimethylammonium or dimethyldiallylammonium salt.

The products sold corresponding to this definition are, more particularly, the products sold as Celquat L 200 and Celquat H 100 by National Starch.

The amphoteric fixing polymers that may be used in accordance with the invention may be selected from polymers comprising units B and C distributed statistically in the polymer chain, where B denotes a unit derived from a monomer comprising at least one basic nitrogen atom and C denotes a unit derived from an acid monomer comprising one or more carboxylic or sulfonic groups, or alternatively B and C may denote groups derived from carboxybetaine or sulfobetaine zwitterionic monomers;

B and C may also denote a cationic polymer chain comprising primary, secondary, tertiary or quaternary amine groups, in which at least one of the amine groups bears a carboxylic or sulfonic group connected via a hydrocarbon group or alternatively B and C form part of a chain of a polymer containing an [alpha],[beta]-dicarboxylic ethylene unit in which one of the carboxylic groups has been made to react with a polyamine comprising one or more primary or secondary amine groups.

The amphoteric fixing polymers corresponding to the definition given above that are more particularly preferred are selected from the following polymers:
(1) copolymers having acidic vinyl units and basic vinyl units, such as those resulting from the copolymerization of a monomer derived from a vinyl compound bearing a carboxylic group such as, more particularly, acrylic acid, methacrylic acid, maleic acid, alpha-chloroacrylic acid, and a basic monomer derived from a substituted vinyl compound containing at least one basic atom, such as, more particularly, dialkylaminoalkyl methacrylate and acrylate, dialkylaminoalkylmethacrylamides and acrylamides. Such compounds are described in patent US 3 836 537.
(2) Polymers comprising units derived from:
   a) at least one monomer selected from acrylamides and methacrylamides substituted on the nitrogen atom with an alkyl group,
   b) at least one acidic comonomer containing one or more reactive carboxylic groups, and
   c) at least one basic comonomer such as esters with primary, secondary, tertiary or quaternary amine substituents of acrylic and methacrylic acids and the product of quaternization of dimethylaminoethyl methacrylate with dimethyl or diethyl sulfate. The N-substituted acrylamides or methacrylamides that are more particularly preferred according to the invention are compounds in which the alkyl groups contain from 2 to 12 carbon atoms and more particularly N-ethylacrylamide, N-*tert*-butylacrylamide, N-*tert-*octylacrylamide, N-octylacrylamide, N-decylacrylamide, N-dodecylacrylamide and the corresponding methacrylamides.

   The acidic comonomers are selected more particularly from acrylic, methacrylic, crotonic, itaconic, maleic and fumaric acid and alkyl monoesters, having 1 to 4 carbon atoms, of maleic or fumaric acid or anhydride.
   The preferred basic comonomers are aminoethyl, butylaminoethyl, N,N'-dimethylaminoethyl and N-*tert*-butylamino ethyl methacrylates.
   The copolymers whose CTFA (4th edition, 1991) name is octylacrylamide/acrylates/butylaminoethyl methacrylate copolymer, such as the products sold as Amphomer® or Lovocryl® 47 by National Starch, are particularly used.
(3) Crosslinked and acylated polyaminoamides partially or totally deriving from polyaminoamides of general formula:

   ⁅CO-R10-CO-Z⁆ (II)

   in which R10 represents a divalent group derived from a saturated dicarboxylic acid, a mono- or dicarboxylic aliphatic acid containing an ethylenic double bond, an ester of a lower alkanol having 1 to 6 carbon atoms of these acids, or a group derived from the addition of any one of said acids to a bis(primary) or bis(secondary) amine, and Z denotes a group derived from a bis(primary), mono- or bis(secondary) polyalkylene-polyamine and preferably represents:
   a) in proportions of from 60 to 100 mol%, the group: 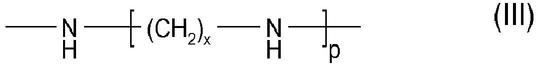 where x = 2 and p = 2 or 3, or alternatively x = 3 and p = 2
      this group being derived from diethylenetriamine, from triethylenetetramine or from dipropylenetriamine;
   b) in proportions of from 0 to 40 mol%, the group (III) above in which x = 2 and p = 1 and which is derived from ethylenediamine, or the group derived from piperazine 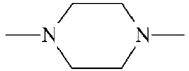
   c) in proportions of from 0 to 20 mol%, the -NH-(CH₂)₆-NH- group being derived from hexamethylenediamine, these polyamino amides being crosslinked by addition reaction of a bifunctional crosslinking agent selected from epihalohydrins, diepoxides, dianhydrides and bis-unsaturated derivatives, using from 0.025 to 0.35 mol of crosslinking agent per amine group of the polyamino amide and acylated by the action of acrylic acid, chloroacetic acid or an alkane sultone, or salts thereof.

   The saturated carboxylic acids are preferably selected from acids having 6 to 10 carbon atoms, such as adipic acid, 2,2,4-trimethyladipic acid and 2,4,4-trimethyladipic acid, terephthalic acid, acids containing an ethylenic double bond such as, for example, acrylic acid, methacrylic acid and itaconic acid.
   The alkane sultones used in the acylation are preferably propane sultone or butane sultone; the salts of the acylating agents are preferably the sodium or potassium salts.
(4) Polymers comprising zwitterionic units of formula: 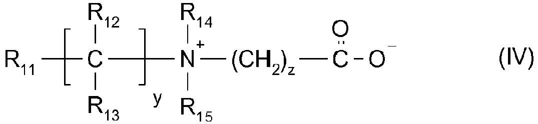 in which R11 denotes a polymerizable unsaturated group such as an acrylate, methacrylate, acrylamide or methacrylamide group, y and z represent an integer from 1 to 3, R12 and R13 represent a hydrogen atom, a methyl, ethyl or propyl group, R14 and R15 represent a hydrogen atom or an alkyl group such that the sum of the carbon atoms in R14 and R15 does not exceed 10.
   The polymers comprising such units may also comprise units derived from non-zwitterionic monomers such as dimethyl- or diethylaminoethyl acrylate or methacrylate or alkyl acrylates or methacrylates, acrylamides or methacrylamides or vinyl acetate.
   By way of example, mention may be made of the methyl methacrylate/methyl dimethylcarboxymethylammonioethyl methacrylate copolymers such as the product sold as Diaformer Z301 by Sandoz;
(5) Polymers derived from chitosan comprising monomer units corresponding to the following formulae: 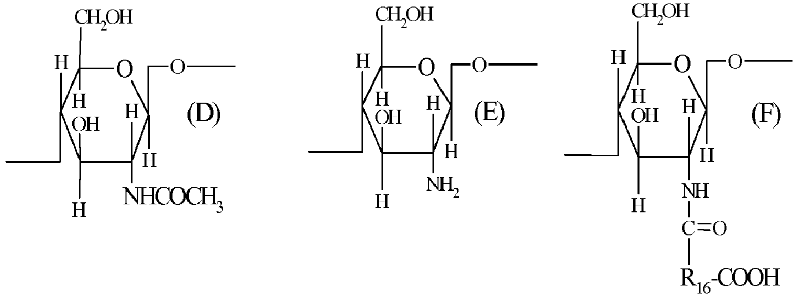 the unit (D) being present in proportions of between 0 and 30%, the unit (E) in proportions of between 5% and 50% and the unit (F) in proportions of between 30% and 90%, it being understood that, in this unit (F), R16 represents a group of formula: 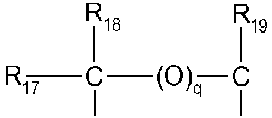 in which, if q = 0, R17, R18 and R19, which may be identical or different, each represent a hydrogen atom, a methyl, hydroxyl, acetoxy or amino residue, a monoalkylamine residue or a dialkylamine residue that are optionally interspersed with one or more nitrogen atoms and/or optionally substituted with one or more amine, hydroxyl, carboxyl, alkylthio or sulfonic groups, an alkylthio residue in which the alkyl group bears an amino residue, at least one of the groups R17, R18 and R19 being, in this case, a hydrogen atom;
   or, if q = 1, R17, R18 and R19 each represent a hydrogen atom, and also the salts formed by these compounds with bases or acids.
(6) Polymers with units corresponding to the general formula (V) are described, for example, in French patent 1 400 366: 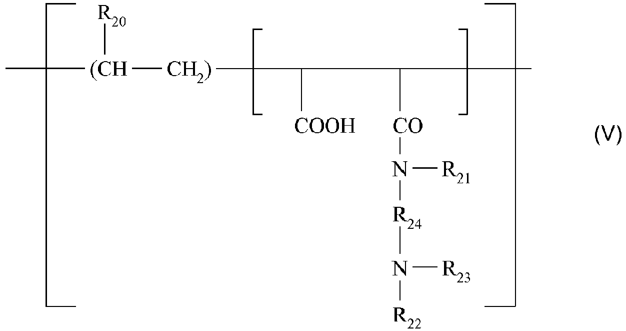 in which R20 represents a hydrogen atom, a CH₃O, CH₃CH₂O or phenyl group, R21 denotes a hydrogen atom or a lower alkyl group such as methyl or ethyl, R22 denotes a hydrogen atom or a C₁-C₆ lower alkyl group such as methyl or ethyl, R23 denotes a C₁-C₆ lower alkyl group such as methyl or ethyl or a group corresponding to the formula: -R24-N(R22)2, R24 representing a group -CH₂-CH₂-, -CH₂-CH₂-CH₂- or -CH₂-CH(CH₃)-, R22 having the meanings mentioned above.
(7) Polymers derived from the N-carboxyalkylation of chitosan, such as N-carboxymethylchitosan or N-carboxybutylchitosan sold as Evalsan by Jan Dekker.
(8) Amphoteric polymers of the type -D-X-D-X selected from:
   a) Polymers obtained by the action of chloroacetic acid or sodium chloroacetate on compounds comprising at least one unit of formula:

      -D-X-D-X-D- (VI)

      where D denotes a 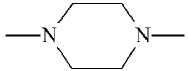 group and X denotes the symbol E or E'; E or E' may be identical or different and denote a divalent group that is an alkylene group with a straight or branched chain containing up to 7 carbon atoms in the main chain, which is unsubstituted or substituted by hydroxyl groups and which may comprise, in addition to oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups.
   b) Polymers having the formula:

      -D-X-D-X- (VII)

      where D denotes a 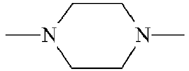 group and X denotes the symbol E or E' and at least once E'; E having the meaning given above and E' is a divalent group that is an alkylene group with a straight or branched chain having up to 7 carbon atoms in the main chain, which is unsubstituted or substituted with one or more hydroxyl groups and containing one or more nitrogen atoms, the nitrogen atom being substituted with an alkyl chain that is optionally interspersed by an oxygen atom and necessarily comprising one or more carboxyl functions or one or more hydroxyl functions and betainized by reaction with chloroacetic acid or sodium chloroacetate.
(9) (C₁-C₅)Alkyl vinyl ether/maleic anhydride copolymers partially modified by semiamidation with an N,N-dialkylaminoalkylamine such as N,N-dimethylaminopropylamine or by semiesterification with an N,N-dialkylaminoalkanol. These copolymers may also comprise other vinyl comonomers such as vinylcaprolactam. Among the amphoteric fixing polymers described above, the ones that are most particularly preferred according to the invention are those of class (3), such as the copolymers whose CTFA name is Octylacrylamide/acrylates/butylamino ethyl methacrylate copolymer, such as the products sold as Amphomer®, Amphomer® LV 71 or Lovocryl® 47 by National Starch and those of class (4) such as the copolymers of methyl methacrylate/methyl dimethylcarboxy-methylammonioethyl methacrylate, sold, for example, as Diaformer Z301 by Sandoz.

The nonionic fixing polymers that may be used according to the present invention are selected, for example, from:
- polyalkyloxazolines;
- vinyl acetate homopolymers;
- vinyl acetate copolymers, for example copolymers of vinyl acetate and of acrylic ester; copolymers of vinyl acetate and ethylene, or copolymers of vinyl acetate and maleic ester, for example dibutyl maleate;
- acrylic ester homopolymers and copolymers, for example copolymers of alkyl acrylates and of alkyl methacrylates, such as the products sold by Rohm & Haas as Primal® AC261 K and Eudragit® NE 30 D, by BASF as 8845, or by Hoechst as Appretan® N9212;
- acrylonitrile copolymers and copolymers of a nonionic monomer selected, for example, from butadiene and alkyl (meth)acrylates; mention may be made of the products sold as CJ 0601 B by Rohm & Haas;
- styrene homopolymers;
- styrene copolymers, for example copolymers of styrene and of alkyl (meth)acrylate, such as the products Mowilith® LDM 6911, Mowilith® DM 611 and Mowilith® LDM 6070 sold by Hoechst, and the products Rhodopas® SD 215 and Rhodopas® DS 910 sold by Rhone Poulenc; copolymers of styrene, of alkyl methacrylate and of alkyl acrylate; copolymers of styrene and of butadiene; or copolymers of styrene, of butadiene and of vinylpyridine;
- polyamides;
- vinyllactam homopolymers such as vinylpyrrolidone homopolymers and such as the polyvinylcaprolactam sold as Luviskol® Plus by BASF; and
- vinyllactam copolymers such as a poly(vinylpyrrolidone/vinyllactam) copolymer sold under the trade name Luvitec® VPC 55K65W by BASF, poly(vinylpyrrolidone/vinyl acetate) copolymers, such as those sold as PVPVA® S630L by ISP, Luviskol® VA 73, VA 64, VA 55, VA 37 and VA 28 by BASF; and poly(vinylpyrrolidone/vinyl acetate/vinyl propionate) terpolymers, for example the product sold as Luviskol® VAP 343 by BASF.

The alkyl groups of the nonionic polymers mentioned above preferably have from 1 to 6 carbon atoms.

According to the invention, it is also possible to use fixing polymers of grafted silicone type comprising a polysiloxane portion and a portion consisting of a non-silicone organic chain, one of the two portions constituting the main chain of the polymer, and the other being grafted onto said main chain.

These polymers are described, for example, in patent applications EP-A-0 412 704, EP-A-0 412 707, EP-A-0 640 105 and WO 95/00578, EP-A-0 582 152 and WO 93/23009 and patents US 4 693 935, US 4 728 571 and US 4 972 037.

These polymers may be amphoteric, anionic or nonionic, and are preferably anionic or nonionic.

Such polymers are, for example, copolymers that may be obtained by free radical polymerization from the monomer mixture formed from:
a) 50 to 90% by weight of *tert*-butyl acrylate;
b) 0 to 40% by weight of acrylic acid;
c) 5 to 40% by weight of a silicone macromer of formula: 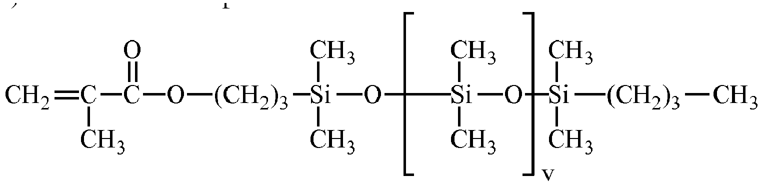 in which v is a number ranging from 5 to 700, the weight percentages being calculated relative to the total weight of the monomers.

Other examples of grafted silicone polymers are, in particular, polydimethylsiloxanes (PDMSs) onto which are grafted, via a thiopropylene-type connecting chain, mixed polymer units of the poly(meth)acrylic acid type and of the polyalkyl (meth)acrylate type and polydimethylsiloxanes (PDMSs) onto which are grafted, via a thiopropylene-type connecting chain, polymer units of the polyisobutyl (meth)acrylate type.

Another type of silicone fixing polymer that may be mentioned is the product Luviflex® Silk sold by BASF.

As fixing polymers, it is also possible to use functionalized or non-functionalized, cationic, nonionic, anionic or amphoteric, silicone or non-silicone polyurethanes, or mixtures thereof.

The polyurethanes used in the present invention are those in particular disclosed in patent applications EP 0 751 162, EP 0 637 600, EP 0 648 485 and FR 2 743 297, of which the Applicant is the Proprietor, and patent applications EP 0 656 021 and WO 94/03510 from BASF and EP 0 619 111 from National Starch.

Polyurethanes particularly suitable in the present invention include the products sold as Luviset PUR® and Luviset® Si PUR by BASF.

The concentration of setting polymer(s) used in the compositions according to the present invention is between 0.1% and 20% and preferably between 0.5% and 10% by weight relative to the total weight of the composition.

The composition according to the invention may comprise other additives such as surfactants, cyclic, linear or branched, silicones that are volatile or involatile, and unmodified or modified with organic groups, having a viscosity from 5×10⁻⁶ at 2.5 m²/s at 25°C, and preferably 1×10⁻⁵ at 1 m²/s.

The silicones that may be used in accordance with the invention may be soluble or insoluble in the composition and in particular may be polyorganosiloxanes that are insoluble in the composition of the invention. They may be in the form of oils, waxes, resins or gums. They may be volatile or involatile.

When they are volatile, the silicones are more particularly selected from those with a boiling point of between 60°C and 260°C.

The silicones as described above may be used, alone or as a mixture, in an amount of between 0.01% and 20% by weight and preferably between 0.1% and 5% by weight.

The compositions of the invention may also comprise non-silicone fatty substances such as mineral, plant, animal and synthetic oils, waxes, fatty esters, ethoxylated or non-ethoxylated fatty alcohols, and fatty acids.

As oils that may be used in the composition of the invention, examples that may be mentioned include:
- hydrocarbon-based oils of animal origin, such as perhydrosqualene;
- hydrocarbon-based oils of plant origin, such as liquid fatty acid triglycerides comprising from 4 to 10 carbon atoms, for example heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, sunflower oil, castor oil, avocado oil, caprylic/capric acid triglycerides, for example those sold by Stéarineries Dubois or those sold as Miglyol® 810, 812 and 818 by Dynamit Nobel, jojoba oil and shea butter oil;
- linear or branched hydrocarbons, of inorganic or synthetic origin, such as paraffin oils, volatile or involatile, and derivatives thereof, Vaseline, polydecenes, hydrogenated polyisobutene such as Parleam®; isoparaffins such as isohexadecane and isodecane; the partially hydrocarbon-based and/or silicone-based fluoro oils, for example those described in document JP-A-2-295 912; fluoro oils that may also be mentioned include perfluoromethylcyclopentane and perfluoro-1,3-dimethylcyclohexane, sold as Flutec® PC1 and Flutec® PC3 by BNFL Fluorochemicals; perfluoro-1,2-dimethylcyclobutane; perfluoroalkanes such as dodecafluoropentane and tetradecafluorohexane, sold as PF 5050® and PF 5060® by 3M, or bromoperfluorooctyl sold as Foralkyl® by Atochem; nonafluoromethoxybutane and nonafluoroethoxyisobutane; perfluoromorpholine derivatives such as 4-trifluoromethyl perfluoromorpholine sold as PF 5052® by 3M;

The wax or waxes are selected in particular from carnauba wax, candelilla wax, esparto grass wax, paraffin wax, ozokerite, plant waxes such as olive wax, rice wax, hydrogenated jojoba wax or the absolute waxes of flowers such as the essential wax of blackcurrant blossom sold by Bertin (France), animal waxes, for example beeswaxes or modified beeswaxes (cerabellina); other waxes or waxy raw materials that may be used according to the invention are especially marine waxes such as the product sold by Sophim under the reference M82, and polyethylene waxes or polyolefin waxes in general.

The saturated or unsaturated fatty acids are more particularly selected from myristic acid, palmitic acid, stearic acid, behenic acid, oleic acid, linoleic acid, linolenic acid and isostearic acid.

The fatty esters are especially carboxylic acid esters, in particular mono-, di-, tri-, or tetracarboxylic esters.

The carboxylic acid esters are especially esters of saturated or unsaturated, linear or branched C1-C26 aliphatic acids and of saturated or unsaturated, linear or branched C1-C26 aliphatic alcohols, the total carbon number of the esters being greater than or equal to 10.

As fatty alcohols, mention may be made of linear or branched, saturated or unsaturated fatty alcohols containing from 8 to 26 carbon atoms, for example cetyl alcohol, stearyl alcohol and the mixture thereof (cetylstearyl alcohol), octyldodecanol, 2-butyloctanol, 2-hexyldecanol, 2-undecylpentadecanol, oleyl alcohol or linoleyl alcohol.

The non-silicone fatty substances in general represent from 0.1 to 50%; preferably from 1 to 30%, and more preferably still from 2 to 20% by weight of the total composition.

A person skilled in the art should know how to add the additives without disturbing the properties of the compositions of the invention.

In addition to the compositions used usually as styling and/or setting compositions, in particular in spray form, through the invention setting products can be used that are not used or not often used in the form of aerosol lacquers for reasons of incompatibility with spraying.

The composition according to the invention may also comprise one or more thickening agents.

For the purposes of the present invention, the term "thickening agent" means an agent capable, by its presence, of increasing the viscosity of the medium by at least 50 centipoise at 25°C and at a shear rate of 1 s⁻¹. Preferably, the thickening agent has, at 1% in water or a 50/50 water/alcohol mixture by weight at 25°C, a viscosity of greater than 100 centipoise at a shear rate of 1 s⁻¹. These viscosities may be measured in particular with viscometers or rheometers with cone-plate geometry.

The thickening and/or gelling agents suitable for the compositions of the invention are well known in the art and may be selected from crosslinked polyacrylic acids, poly(oxyalkylene)glycols, poly(oxyalkylene)glycol esters, alginates, biosaccharides, starches and derivatives thereof such as distarch phosphate and carboxymethylstarch, natural gums such as xanthane, guar, carouba gums, scleroglucans, chitin and chitosan derivatives, carraghenans, modified or unmodified celluloses such as hydroxypropyl guar gum, methylhydroxyethylcellulose, hydroxypropylmethyl cellulose and hydroxyethyl cellulose, clays, and mixtures thereof.

By way of example of gelling agents, especially that are in the aqueous phase, mention may be made of Sepigel® 305 sold by Seppic, Fucogel® 1000 PP sold by Solabia, Synthalen® K sold by 3VSA, Hostacerin® AMPS sold by Clariant, Lubragel® MS sold by Guardian, Satiagel® KSO sold by Degussa and Keltrol® sold by Kelco, Salcare SC 95 or SC 96 sold by Ciba.

The concentration of thickening agents and/or gelling agents may vary from 0.01 to 4%, and preferably from 0.1 to 2% by weight relative to the total weight of the composition.

The compositions used in the invention may also comprise cosmetically acceptable adjuvants, such as for example penetrants, fragrances, dyes, plasticizers, buffers, and various typical adjuvants such as ceramides, pseudoceramides, vitamins or provitamins such as panthenol, opacifiers, reducing agents, preservatives, mineral fillers, pearlescent agents, flakes, sunscreens, proteins, moisturizers, emollients, demulcents, antifoaming agents, antiperspirants, free-radical scavengers, bactericides, sequestrants, antidandruff agents, antioxidants, basifying agents, acidifying agents and any other additive conventionally used in cosmetic compositions intended to be applied to hair.

According to another of its aspects, a subject of the present invention is a hair styling device as defined in claim 6 of the present application.

### Support

The support may be rigid, semi-rigid or flexible.

The support comprises or is preferably made of a non-absorbent material. "Non-absorbent" is understood to mean intrinsically impermeable to the composition, i.e. not allowing diffusion of the composition inside it nor the destruction of packets or droplets of composition.

The non-absorbent material may be a thermoplastic or metallic material, in particular selected from polyolefins, polyamides including nylons, polyester, natural fibers, stainless steel, ceramics.

The support may be constituted by or comprise an applicator portion, for example a flexible applicator portion, of relatively low thickness. The applicator portion is preferably crossed through by the cavities, which are separated by partitions made of a preferably non-absorbent material, for example thermoplastic or metallic. In some other embodiments the applicator portion comprises studs (also called pins) or other portions in relief that include the cavities. The cavities may be situated at the tip end of the portions in relief, for example one cavity forming a hollow tip at the end of a portion in relief. The length of the portions in relief may be comprised between.2 and.200mm. The hollow tip may have a round shape, whose concavity is directed outwards. The applicator portion can hold droplets of the composition to be applied. To allow the transfer of the droplets of composition onto the hair, the applicator portion may be directed to the hair, even put in contact with the hair, as mentioned previously.

The applicator portion may be selected from the following list: a mesh, a grid in particular a molded grid, a perforated membrane, in particular a perforated nonwoven, a low-thickness foam obtained for example by slicing a cellular material, a woven, a perforated film, in particular plastic or metallized, a network of studs.

The support may be translucent, being in particular constituted of a grid whose mesh can be seen through, specifically comprising mesh with width between 100 µm and 5 mm.

The applicator portion, even the support, may be vinyl-based, PVA- or PVP-based, pseudolatex such as acrylic polymers, polyurethanes, latex elastomers, this list being non-limiting.

The cavities may be larger with opening less than or equal to 5 mm, preferably between 10 µm and 3 mm, even between 100 µm and 2 mm.

The cavities may all have the same dimensions for a given support. The dimensions of the cavities may be adapted to the rheology and surface tension of the composition.

As indicated previously, the number of cavities may vary from 1 to 100,000, preferably from 50 to 50,000, more preferably from 100 to 5000, the cavities extending for example on a surface area of the support varying from 1 to 1000 cm², preferably from 5 to 500 cm².

As a variant, the distribution and/or the size of the cavities is irregular, in particular when depositing the composition according to a specific arrangement is the aim.

The cavities are for example made by perforating a membrane according to a specific arrangement. In one example, the applicator portion receives a treatment, in particular a selective printing by screen printing for example, to close, optionally partially, cavities around arrangements to be made. Such irregularity in the distribution of cavities is interesting for some hair styles, for which different setting for different tresses is the aim.

The support may comprise a frame arranged at least partially around the applicator portion and preferably all the way around. The frame may be rigid, semi-rigid or flexible. The frame may comprise a synthetic material, in particular a thermoplastic elastomer or a foam with closed cells, in particular NBR or SBR or a natural or synthetic rubber.

Preferably, the frame is more rigid than the applicator portion and can hold it in a specific shape, for example measurably planar.

The frame may be fixed to the applicator portion by any suitable means, in particular by gluing, heat-sealing, crimping. The applicator portion may be made by molding or overmolding with the frame, in the same material or in a different material.

The device may comprise a flexible membrane, and more generally any intermediate part between the user and the applicator portion, extending behind the applicator portion. This membrane or intermediate portion is for example made of a soft, non-absorbent material, impermeable to the hair care composition in liquid form.

The frame and/or the membrane or intermediate portion may define a gripping surface for the device, and in particular of the support.

The presence of a frame and/or a membrane or intermediate portion impermeable to the composition may make it easier to handle the support, particularly when it is reloaded with composition and, when it is put on the hair, fingers are not in contact with the composition, which may stain.

Preferably, the support is reloaded with composition before each use. For this purpose, the device may comprise a container containing the composition. For example, the container may be a case housing a sponge soaked with composition, that is accordingly available to load the applicator portion, which is for example in the form of a mesh or grid. The applicator portion then has to be pressed onto the sponge to load it with composition.

The invention is not limited to a specific container to contain the composition that must load the support.

The device may comprise several supports, differing by the size and/or distribution of cavities, in particular their surface density. The user may then select, for a specific use, in particular shaping, styling, hair design, setting or touch ups, the best suited support.

The support may also be preloaded with composition. In this case, the support is supplied to the user preloaded with composition, for example in a sealed packaging.

As a variant, the support, preloaded or not, is presented as a roll, with optional perforation to make it easier to separate the sheets of support. The sheets may be for single use.

In a specific embodiment, the support is presented as a pile or roll of sheets each defining an applicator portion prefilled with the composition to be applied. This method of distribution is particularly interesting in a professional setting; it is hygienic and means for example that a distribution of the composition on the hair can be reproduced easily according to a specific pattern defined as a function of the targeted hair style. In the presence of active cavities, these may be controlled according to a pattern that is to be made on the hair.

The pile or roll of sheets may be submerged, if need be, in a container that contains the composition to be applied. As a variant, the sheets are soaked with the composition without being submerged in it.

According to another of its aspects, a subject of the present invention is a hair styling system as defined in claim 7 of the present application. The support may comprise a flexible or non flexible application part.

The support may comprise an application part comprising studs or other portions in relief.

The invention delivers a perfectly directional hair styling or makeup system, without losses or stains.

The hair styling or makeup system comprises for example one or more stems that are attached at one end to the gas blowing system, for example an aerosol can, and at the other end to the support. These stems are for example molded of a single part with a support frame. The stem length is selected so that the gas jet emitted by the gas blowing system forces the droplets of composition to leave the cavities in the direction of the hair. The aerosol can may contain compressed air and be recharged.

According to another of these aspects, the subject of the present invention is also a process of hair styling or makeup using a device or a hair styling or makeup system as described previously.

The invention may be better understood from reading the following detailed description of non-limiting implementation examples thereof, and with reference to the attached drawing, in which:
- figures 1A and 1B illustrate a hair styling process according to the invention.
- figures 2A to 2F are different views of supports according to the invention,
- figures 3B and 3F are cross sections along IIIB of figure 2B and along III F of figure 2F,
- figures 3G shows a detail of figure 3F,
- figure 4 shows a cross section of a device according to the invention,
- figure 5 shows in perspective a hair styling system according to the invention,
- figure 6 illustrates a hair styling process using the system shown in figure 5, and
- figure 7 shows a cross section of a variant of embodiment for the support according to the invention.

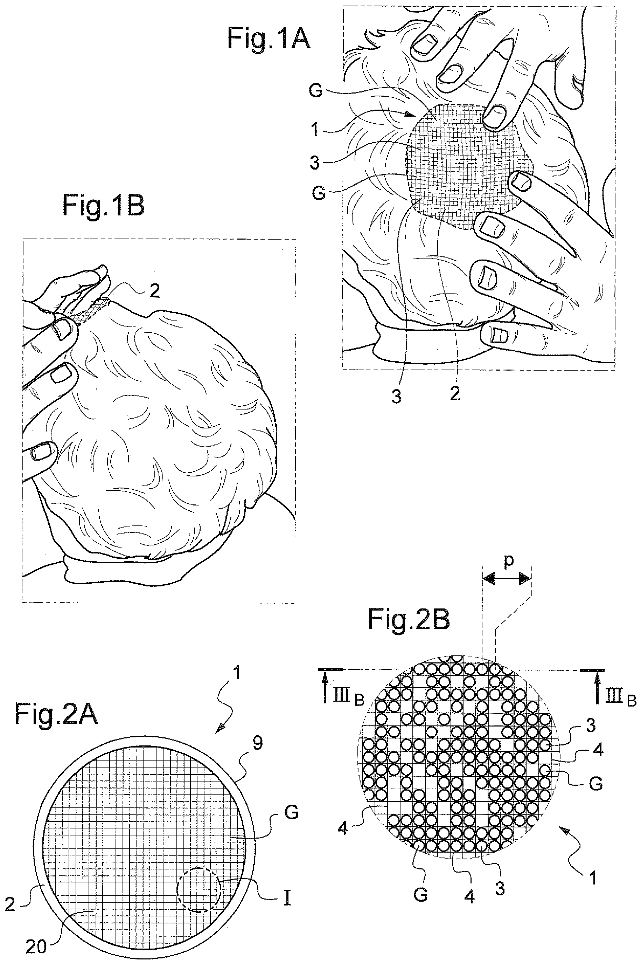

The process according to the invention may be implemented directly by the consumer, to position or touch up his or her hair. In an alternative way, it can be used in a professional setting, as illustrated for example in figures 1A and 1B.

Figure 1A represents a support 2 according to the invention not comprising a frame, in the form of a mesh of non-absorbent material, for example of nylon, holding droplets G of a composition P containing a setting agent, that the hair stylist has placed on the hair.

The hair stylist may facilitate the transfer of droplets onto the hair using an air jet, for example a cold air jet produced by an aerosol can filled with compressed air, not shown.

Figure 1B shows that the hair stylist, after having reloaded the support with the composition, may repeat the operation by placing the mesh on another part of the hair.

In the example of the device 1 illustrated in figure 2A, the support 2 comprises a plurality of cavities 3 in which droplets G of the composition to be applied are held.

In this variant, the support 2 comprises an applicator portion 20 formed of a metal grid, held by a frame 9.

The shape of the cavities and the nature of the support govern the size of the drops, how many there are, how they transfer and how they are spaced once deposited on the hair.

Figure 2B shows a zoom of the detail of area I of figure 2A. When the cavities are close together, for example with a step p between the mesh between 10 µm and 300 µm as in the example illustrated in figures 2A and 2B, droplets G are very numerous and may agglomerate on hair. This type of support is interesting in particular to obtain strong setting, which lasts a long time.

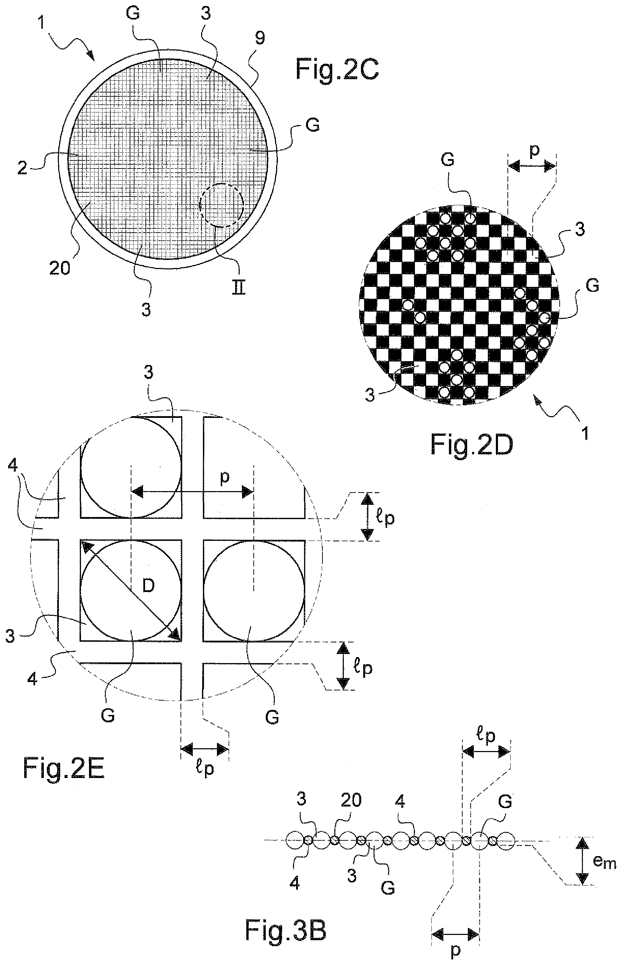

According to the type of shaping targeted, one may want a more natural effect and be interested in using a configuration where the cavities 3 are spaced further apart, for example with a step p between the mesh between 300 µm and 3 mm as illustrated in figures 2C and 2D, which represents the detail of area II in figure 2C, to limit the risks of agglomeration and give a lighter style with fewer droplets G.

Figure 2E shows that each cavity 3 may be defined by a plurality of interconnected partitions 4, extending in multiple directions. Each cavity may be defined for example when observed face on by a succession of four partitions 4 or more, each measurably rectilinear, forming a loop. The partitions may be formed by the filaments of a grid as in the examples illustrated in figures 2A-2D.

In cross section the cavities may present any shape, in particular a round, square, rectangular, hexagonal or polygonal shape.

The largest opening dimension D is preferably less than or equal to 2 mm.

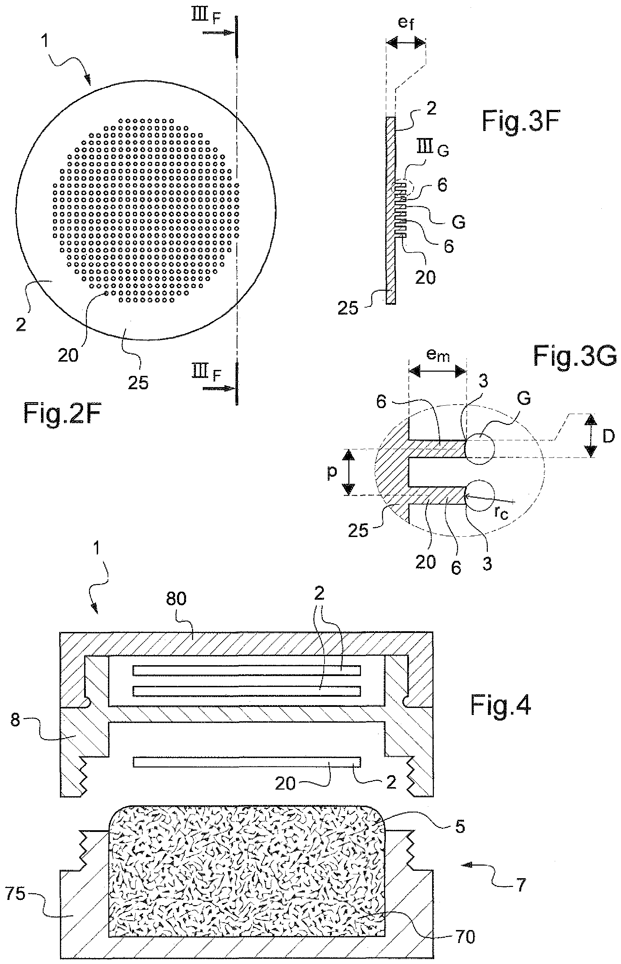

Figure 3B illustrates in cross section IIIB the applicator portion 20 of the support 2 of figure 2B presented in the form of a grid, in which the cavities 3 are separated by partitions 4 formed by the threads of the grid. The diameter of the threads corresponds in the case of a grid to the width lp of the partitions 4 defining the cavities and to the thickness eₘ of the applicator portion 20.

Thickness eₘ of the applicator portion 20 may lies for example between 10 µm and 5 mm.

The droplets G are held by capillary action in the cavities 3.

The device 1 illustrated in figure 2F comprises an application portion 20 formed by a plurality of portions in relief (e.g. studs) with a hollow tip forming a cavity 3 to hold a droplet G of the composition before application. Preferably the portions in relief are elongate in shape, for example cylindrical, as illustrated in Figure 3G, or conical. In the variant shown, opening size D of cavities 3 equals 1.5mm, the applicator portion 20 consists of a network of studs molded on an intermediate part 25 serving as a gripping surface for the device 1. The applicator portion 20 of the illustrated device has a disk shape of about 10 cm in diameter, the intermediate part 25 being about 14 cm in diameter and 5mm in thickness e_{f}. The height of the portions in relief, corresponding to the thickness eₘ of the applicator portion 20, is 5mm in the illustrated embodiment. Other embodiments may comprise longer studs forming application portion with a thickness eₘ lying for example between 2mm and 200 mm. In other examples, cross sections of the portions in relief may present any shape, in particular a round, square, rectangular, hexagonal or other polygonal shape. Cavities 3, formed at the end of the portions in relief, have for example a concavity of a radius between 0.2 and 4 mm, for example.

The device may comprise more than 100 pins or studs. The pins or studs may all have a same height, as shown. The network may be regular (i.e. with same step p' between two studs), as shown, or irregular with varying step.

To use the device of figure 2F, one puts the tips into contact with a liquid composition. Packets (droplets) form at the tips of the portions in relief, and are hold by the cavities 3. Then the portions in relief are brought into contact with hair. The packets transfer onto the hair. After drying, the packets provide hair setting.

Figure 4 shows a case 7 comprising a cover 8 and a container 70 formed of a sponge 5 containing the composition P to be applied.

The support 2 is in the example illustrated formed of an applicator portion 20 in the form of a disk about 6 cm in diameter that treats a quarter of the hair after having been loaded once. The support 2 may be placed on the hair and tapped lightly, which deposits the drops of product on the hair.

The gesture is very feminine since it recalls the powder puff used to powder one's face. This embodiment is suited to setting and "touching up" the hair all day long.

A same device 1 may comprise several distinct supports 2, for example grids presenting different meshes as in the example illustrated.

A first support 2 may be arranged in the base 75 of the case 7, above the sponge 5 when it is not used.

The example illustrated further comprises two extra supports 2, stored when they are not used in a housing 85 of the cover 8 of the case 7. The housing 85 is closed by a closure lid capsule 80.

The cover 8 may screw onto the base 75 of the case to close it. Any closure system known to a person skilled in the art, both for the cover 8 and for the capsule 80, may be used.

In a variant that is not illustrated, the supports comprise an applicator portion and an intermediate portion that is impermeable to composition P defining a gripping surface that makes handling them easier.

In another variant not shown, the cover 8 does not comprise a housing and the device 1 comprises a single support.

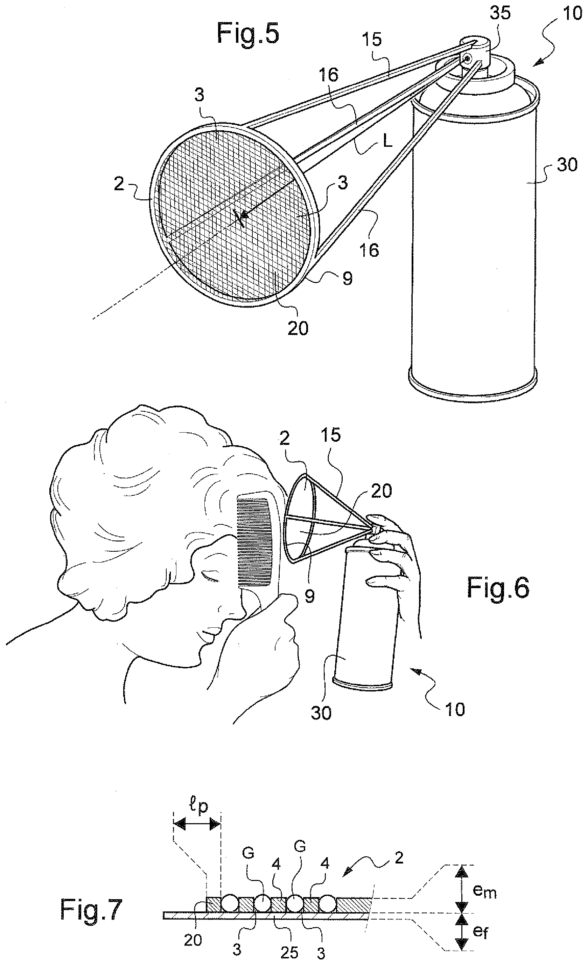

Figure 5 illustrates a hair styling system 10 comprising a support 2 connected by an attachment system 15 to a gas source 30 constituted of a compressed air can. The attachment system 15 illustrated comprises three stems 16 connecting the frame 9 of the support 2 to the nozzle 35 of the source 30. The stems 16 hold the support 2 at a distance L from the nozzle 35. The person skilled in the art may select any other suitable attachment system without exiting the scope of the invention.

The distance L between the gas outlet nozzle 35 and the support 2 is for example between 4 and 30 cm.

Figure 6 illustrates a user using the hair styling system 10 according to figure 5.

The user shapes his or her hair using a comb while using the system 10, and accordingly easily sets his or her hair tress by tress. He or she reloads the applicator portion 20 from a container that is not shown.

The support illustrated on figure 7 comprises an applicator portion 20 of thickness eₘ and an intermediate portion 25 of thickness e_{f} defining a gripping surface, impermeable to the composition P, but permeable to air so that, if it is desired, an air jet may be used to make the transfer of droplets G from cavities 3 onto the hair easier.

In the example illustrated in figure 7, the gripping surface 25 extends further on the sides of the applicator portion 20, thus making handling easier.

The invention is not limited to the examples illustrated.

A process of making up not covered by the claims may for example use an adhesive composition and allow making up with the transfer of sticky droplets then application of particles or other compounds that stick to the skin by virtue of the droplets.

In a variant the process comprises the transfer of droplets of a colored composition that forms "pixels" or dots with a distribution that allows arrangements or gradients to be made on the body, in particular the face, the cheeks for example or to create pixelated effects for example on the lips.

## Claims

1. A process for styling hair, comprising transferring onto hair, packets, preferably droplets (G), of a hair care composition (P), held by capillary action or otherwise in cavities (3) in a support (2), by putting said support (2) in contact with keratinous substances or mucous membranes and/or by ejecting said packets, preferably said droplets, from the support (2) by mechanical action and/or by using a gas jet, preferably coming from a container of compressed air, the composition comprising at least a setting agent, the composition comprising one or more fixing polymers as the setting agent(s).

2. The process as claimed in the previous claim, the support (2) being constituted of a non-absorbent substance.

3. The process as claimed in claims 1 and 2, the support cavities crossing through or being formed at the end of portions in relief (6).

4. The process as claimed in any one of the previous claims, comprising the prior step consisting in: loading the support (2) with the composition (P), in particular by direct sampling via the support (2) from a container (70) containing the composition, in particular the support being pressed onto a sponge (5) soaked with composition, so as to be loaded with composition.

5. The process as claimed in any one of the previous claims, where the ejection of droplets occurs upon demand and results from a thermal effect or use of a piezoelectric element.

6. A hair styling device (1) comprising:
i. a support (2) comprising a applicator portion (20) comprising a plurality of cavities (3) that can hold packets by capillary action or otherwise, preferably droplets (G), of a liquid composition,
ii. packets, preferably droplets, of a hair care composition (P) comprising at least one setting agent, the composition comprising one or more fixing polymers as the setting agent(s), held by capillary action or otherwise in the cavities (3) of the support (2), the applicator portion (20) being flexible and/or presenting portions in relief (6) comprising said cavities (3), in particular cavities forming hollow tips of portions in relief.

7. A hair styling system (100) comprising:
i. a support (2) comprising a applicator portion (20) comprising a plurality of cavities (3) that can hold packets by capillary action or otherwise, preferably droplets (G), of a composition, the composition comprising at least a setting agent, the composition comprising one or more fixing polymers as the setting agent(s), the applicator portion (20) being flexible and/or presenting portions in relief (6) comprising said cavities (3),
ii. a system for blowing gas (30), in particular air,
iii. a setting system (15) for the support (2) on the gas blowing system (30), the gas blowing on the support causing the ejection and/or transfer of packets, preferably droplets, of the support toward the area to be treated.

8. The device as claimed in claim 6, or system as claimed in claim 7, the support (2) being constituted of a non-absorbent substance.

9. The device as claimed in either of claims 6 and 8, or system as claimed in either of claims 7 and 8, the cavities (3) having a larger opening size (D) less than or equal to 2 mm, preferably comprised between 1 µm and 2 mm.

10. The device as claimed in any one of claims 6, 8 or 9, or system as claimed in any one of claims 7 to 9, the support (2) comprising from 1 to 100,000, preferably from 50 to 50,000, most preferably from 100 to 5,000 cavities.

11. The device as claimed in any one of claims 6, 8, 9 or 10, or system as claimed in any one of claims 7 to 10, where the cavities extend on a surface area of the support (2) varying from 1 to 1,000 cm², preferably from 5 to 500 cm².

12. The device as claimed in any one of claims 6, 8, 9, 10 or 11, or system as claimed in any one of claims 7 to 11, further comprising a container (70) containing the composition (P), in particular a sponge (5) soaked with composition.

13. The device as claimed in any one of claims 6, 8, 9, 10, 11 or 12, or system as claimed in any one of claims 7 to 12, the support (2) being configured to store the droplets of composition and eject them upon demand, in particular by a thermal effect or using a piezoelectric element.

14. The device as claimed in any one of claims 6, 8, 9, 10, 11, 12 or 13, or system as claimed in any one of claims 7 to 13, the support (2) comprising a frame.

## Patentansprüche

1. Verfahren zum Stylen von Haar, umfassend Überführen von Packungen, bevorzugt Tropfen (G), von einer Haarpflegezusammensetzung (P), gehalten durch Kapillarwirkung oder anderweitig in Kavitäten (3) in einem Träger (2), auf Haar durch Bringen des Trägers (2) in Kontakt mit keratinösen Substanzen oder Schleimhäuten und/oder durch Ausstoßen der Packungen, bevorzugt der Tropfen, von dem Träger (2) durch mechanische Wirkung und/oder durch Verwenden eines Gasstrahls, bevorzugt ausgehend von einem Behälter mit komprimierter Luft, wobei die Zusammensetzung mindestens ein Festigungsmittel umfasst, wobei die Zusammensetzung ein oder mehr Fixierungspolymere als das/die Festigungsmittel umfasst.

2. Verfahren wie im vorhergehenden Anspruch beansprucht, wobei der Träger (2) aus einer nicht-absorbierenden Substanz besteht.

3. Verfahren wie in den Ansprüchen 1 und 2 beansprucht, wobei die Trägerkavitäten hindurchgehend sind oder geformt sind am Ende von Reliefanteilen (6).

4. Verfahren wie in einem der vorhergehenden Ansprüche beansprucht, umfassend den vorherigen Schritt, der besteht aus: Beladen des Trägers (2) mit der Zusammensetzung (P), insbesondere durch direktes Probeaufnehmen über den Träger (2) von einem Behälter (70), der die Zusammensetzung enthält, wobei der Träger insbesondere auf einen Schwamm (5), der mit Zusammensetzung durchtränkt ist, gepresst wird, um so mit Zusammensetzung beladen zu werden.

5. Verfahren wie in einem der vorhergehenden Ansprüche beansprucht, wobei das Ausstoßen von Tropfen nach Bedarf stattfindet und aus einer thermischen Wirkung oder Verwendung eines piezoelektrischen Elements resultiert.

6. Vorrichtung zum Stylen von Haar (1), umfassend:
i. einen Träger (2), umfassend einen Aufbringungsanteil (20), umfassend eine Vielzahl von Kavitäten (3), die Packungen durch kapillare Wirkung oder anderweitig halten können, bevorzugt Tropfen (G), von einer flüssigen Zusammensetzung,
ii. Packungen, bevorzugt Tropfen, von einer Haarpflegezusammensetzung (P), umfassend mindestens ein Festigungsmittel, wobei die Zusammensetzung ein oder mehr Fixierungspolymere als das(die) Festigungsmittel umfasst, gehalten durch kapillare Wirkung oder anderweitig in den Kavitäten (3) von dem Träger (2), wobei der Aufbringungsanteil (20) flexibel ist und/oder Reliefanteile (6), umfassend die Kavitäten (3), insbesondere Kavitäten mit hohlen Spitzen von Reliefanteilen, bereitstellt.

7. System zum Stylen von Haar (100), umfassend:
i. einen Träger (2), umfassend einen Aufbringungsanteil (20), umfassend eine Vielzahl von Kavitäten (3), die Packungen durch kapillare Wirkung oder anderweitig halten können, bevorzugt Tropfen (G), von einer Zusammensetzung, wobei die Zusammensetzung mindestens ein Festigungsmittel umfasst, wobei die Zusammensetzung ein oder mehr Fixierungspolymere als das/die Festigungsmittel umfasst, wobei der Aufbringungsanteil (20) flexibel ist und/oder Reliefanteile (6), umfassend die Kavitäten (3), bereitstellt,
ii. ein System zum Treiben von Gas (30), insbesondere Luft,
iii. ein Anordnungssystem (15) für den Träger (2) auf dem System zum Treiben von Gas (30), wobei das Treiben von Gas auf dem Träger das Ausstoßen und/oder das Überführen von Packungen, bevorzugt Tropfen, von dem Träger auf den zu behandelnden Bereich verursacht.

8. Vorrichtung wie in Anspruch 6 beansprucht oder System wie in Anspruch 7 beansprucht, wobei der Träger (2) aus einer nicht-absorbierenden Substanz besteht.

9. Vorrichtung wie in einem der Ansprüche 6 und 8 beansprucht oder System wie in einem der Ansprüche 7 und 8 beansprucht, wobei die Kavitäten (3) eine größere Öffnungsgröße (D) von kleiner als oder gleich 2 mm, bevorzugt zwischen 1 µm und 2 mm, aufweisen.

10. Vorrichtung wie in einem der Ansprüche 6, 8 oder 9 beansprucht oder System wie in einem der Ansprüche 7 bis 9 beansprucht, wobei der Träger (2) 1 bis 100.000, bevorzugt 50 bis 50.000, am stärksten bevorzugt 100 bis 5.000 Kavitäten umfasst.

11. Vorrichtung wie in einem der Ansprüche 6, 8, 9 oder 10 beansprucht oder System wie in einem der Ansprüche 7 bis 10 beansprucht, wobei sich die Kavitäten auf einer Oberfläche des Trägers (2), die von 1 bis 1.000 cm², bevorzugt von 5 bis 500 cm² reicht, erstrecken.

12. Vorrichtung wie in einem der Ansprüche 6, 8, 9, 10 oder 11 beansprucht oder System wie in einem der Ansprüche 7 bis 11 beansprucht, ferner umfassend einen Behälter (70), der die Zusammensetzung (P), insbesondere einen Schwamm (5), der mit der Zusammensetzung durchtränkt ist, enthält.

13. Vorrichtung wie in einem der Ansprüche 6, 8, 9, 10, 11 oder 12 beansprucht oder System wie in einem der Ansprüche 7 bis 12 beansprucht, wobei der Träger (2) zum Speichern der Tropfen der Zusammensetzung konfiguriert ist und sie bei Bedarf ausstößt, insbesondere durch eine thermische Wirkung oder unter Verwendung eines piezoelektrischen Elements.

14. Vorrichtung wie in einem der Ansprüche 6, 8, 9, 10, 11, 12 oder 13 beansprucht oder System wie in einem der Ansprüche 7 bis 13 beansprucht, wobei der Träger (2) einen Rahmen umfasst.

## Revendications

1. Procédé de coiffage, comprenant le transfert sur les cheveux, de paquets, de préférence de gouttelettes (G), d'une composition de soin capillaire (P), retenus par capillarité ou autrement dans des cavités (3) d'un support (2), en mettant en contact ledit support (2) avec des matières kératiniques ou des muqueuses et/ou en éjectant lesdits paquets, de préférence lesdites gouttelettes, du support (2) par une action mécanique, et/ou à l'aide d'un jet de gaz, provenant de préférence d'un récipient d'air comprimé, la composition comprenant au moins un agent fixant, la composition comprenant un ou plusieurs polymères fixateurs en tant qu'agent(s) fixant(s).

2. Procédé selon la revendication précédente, le support (2) étant constitué d'une matière non absorbante.

3. Procédé selon les revendications 1 et 2, les cavités du support étant traversantes ou étant formées à l'extrémité de parties en relief (6).

4. Procédé selon l'une quelconque des revendications précédentes, comprenant l'étape préalable consistant à :charger le support (2) avec la composition (P), notamment par prélèvement direct par le support (2) à partir d'un récipient (70) contenant la composition, en particulier le support étant pressé sur une éponge (5) imbibée de composition, de sorte à se charger en composition.

5. Procédé selon l'une quelconque des revendications précédentes, où l'éjection des gouttelettes se produit à la demande et résulte d'un effet thermique ou de l'utilisation d'un élément piézo-électrique.

6. Dispositif de coiffage (1) comprenant :
i. un support (2) comprenant une partie applicatrice (20) comprenant une pluralité de cavités (3) aptes à retenir par capillarité ou autrement des paquets, de préférence des gouttelettes (G), d'une composition fluide,
ii. des paquets, de préférence des gouttelettes, d'une composition de soin capillaire (P) comprenant au moins un agent fixant, la composition comprenant un ou plusieurs polymères fixateurs en tant qu'agent(s) fixant(s), retenus par capillarité ou autrement dans les cavités (3) du support (2), la partie applicatrice (20) étant souple et/ou présentant des parties en relief (6) comprenant lesdites cavités (3), en particulier des cavités formant des pointes creuses de parties en relief.

7. Système de coiffage (100) comprenant :
i. un support (2) comprenant une partie applicatrice (20) comprenant une pluralité de cavités (3) aptes à retenir par capillarité ou autrement des paquets, de préférence des gouttelettes (G) d'une composition, la composition comprenant au moins un agent fixant, la composition comprenant un ou plusieurs polymères fixateurs en tant qu'agent(s) fixant(s), la partie applicatrice (20) étant souple et/ou présentant des parties en relief (6) comprenant lesdites cavités (3),
ii. un système de soufflage de gaz (30), notamment d'air,
iii. un système de fixation (15) du support (2) sur le système de soufflage de gaz (30), le soufflage du gaz sur le support provoquant l'éjection et/ou le transfert de paquets, de préférence de gouttelettes, du support vers la zone à traiter.

8. Dispositif selon la revendication 6, ou système selon la revendication 7, le support (2) étant constitué d'une matière non absorbante.

9. Dispositif selon l'une des revendications 6 et 8, ou système selon l'une des revendications 7 et 8, les cavités (3) ayant une plus grande dimension d'ouverture (D) inférieure ou égale à 2 mm, de préférence, comprise entre 1 µm et 2 mm.

10. Dispositif selon l'une quelconque des revendications 6, 8 ou 9, ou système selon l'une quelconque des revendications 7 à 9, le support (2) comprenant de 1 à 100.000, de préférence, de 50 à 50000, plus préférablement, de 100 à 5000 cavités.

11. Dispositif selon l'une quelconque des revendications 6, 8, 9 ou 10, ou système selon l'une quelconque des revendications 7 à 10, où les cavités s'étendent sur une surface du support (2) variant de 1 à 1000 cm², de préférence, de 5 à 500 cm².

12. Dispositif selon l'une quelconque des revendications 6, 8, 9, 10 ou 11, ou système selon l'une quelconque des revendications 7 à 11, comprenant en outre un récipient (70) contenant la composition (P), notamment une éponge (5) imbibée de composition.

13. Dispositif selon l'une quelconque des revendications 6, 8, 9, 10, 11 ou 12, ou système selon l'une quelconque des revendications 7 à 12, le support (2) étant configuré pour conserver des gouttelettes de composition et les éjecter à la demande, notamment par un effet thermique ou à l'aide d'un élément piézo-électrique.

14. Dispositif selon l'une quelconque des revendications 6, 8, 9, 10, 11, 12 ou 13, ou système selon l'une quelconque des revendications 7 à 13, le support (2) comprenant un cadre.
